# EUROPEAN PATENT APPLICATION

(11) **EP 2 657 867 A1**
(43) Date of publication of application: **30.10.2013**
(21) Application number: 12186046.4
(22) Date of filing: 26.09.2012
(51) Int. Cl.: G06F 19/00

(54) **Digital image storage system and human body data matching method for medical cosmetic applications**

(30) Priority: 23.04.2012 TW 101114334; 16.08.2012 TW 101129679
(71) Applicant: Chen, Chun-Leon, New Taipei City 24764 (TW)
(72) Inventor: Chen, Chun-Leon, 24764 New Taipei City (TW); Cha, Jennifer, Las Vegas, NV 89146 (US)
(74) Representative: Ruschke, Hans Edvard

(57) **Abstract**

A human body data matching method for medical cosmetic applications includes the step of providing a human body imaging system (1) having a digital image pickup module (11) for scanning and photographing human bodies to obtain image data, the step of rendering the image data into human digital images, the step of storing the human digital images in a databank (21), the step of fetching pre-stored storage human digital images of the person going to take an orthopedic or cosmetic surgery from the databank (21), and the step of matching the fetched human digital images to make sure the location and differences. This method prevents surgical errors due to misunderstanding in communication between the surgeon and the patient.

## Description

### BACKGROUND OF THE INVENTION

### 1. Field of the Invention:

The present invention relates to medical cosmetic technology and more particularly, to a digital image storage system for medical cosmetic applications. The invention relates also to a human body data matching method using this digital image storage system.

### 2. Description of the Related Art:

There are some medical institutions to provide storage banks for storing stem cells, sperm, egg, umbilical cord blood and other medical-related items for future needs.

However, these medical-related storage items are taken from human bodies and can only be used for a particular purpose.

However, these storage banks contribute nothing to some orthopedic surgery or cosmetic surgery.

Many medical surgeries and cosmetic surgeries are taken in order to make specific parts of the bodies of the patients back to the best body conditions, such as skin color, body proportions, facial length, etc. However, patients going to take a surgery may be unable to describe their best body conditions accurately, causing an error in communication with the surgeon and leading to surgery dissatisfaction.

Therefore, the invention discusses an effective method for enabling a surgeon to match personal early reference data rapidly before performing a surgery.

### SUMMARY OF THE INVENTION

The present invention has been accomplished under the circumstances in view. It is one object of the present invention to provide a human body data matching method, which comprises a first step of providing a human body imaging system comprising a digital image pickup module for scanning human bodies of clients synchronously based on a reference point to obtain image data of the soft tissues and hard tissues and skin tone appearance of predetermined parts of the physical structure of the human bodies, a second step of rendering the image data thus obtained into human digital images, a third step of storing the human digital images in a databank, a fourth step of fetching pre-stored storage human digital images of the patient going to take an orthopedic or cosmetic surgery from the databank for future reconstruction or study of how individual growth pattern, or for forensic or recreational purposes prior to performing the orthopedic or cosmetic surgery, and a fifth step of matching the fetched human digital images to make sure the location and differences.

To achieve this and other objects of the present invention, a digital image storage system comprises a human body imaging system for scanning and photographing the human bodies of persons during their optimal lifetime synchronously based on a reference point to obtain image data of the soft tissues and hard tissues of predetermined parts of the physical structure of the human bodies and the related skin tone appearance and to render the image data thus obtained into human digital images, a data storage device electrically connected to the human body imaging system for receiving and storing the human digital images, and a display device electrically connected to the data storage device for displaying the human digital images.

Other advantages and features of the present invention will be fully understood by reference to the following specification in conjunction with the accompanying drawings, in which like reference signs denote like components of structure.

### BRIEF DESCRIPTION OF THE DRAWINGS

FIG. 1 illustrates a system block diagram of digital image storage system for medical cosmetic applications in accordance with the present invention.
FIG. 2 is a flow chart of a human body data matching method in accordance with the present invention.

### DETAILED DESCRIPTION OF THE PREFERRED EMBODIMENT

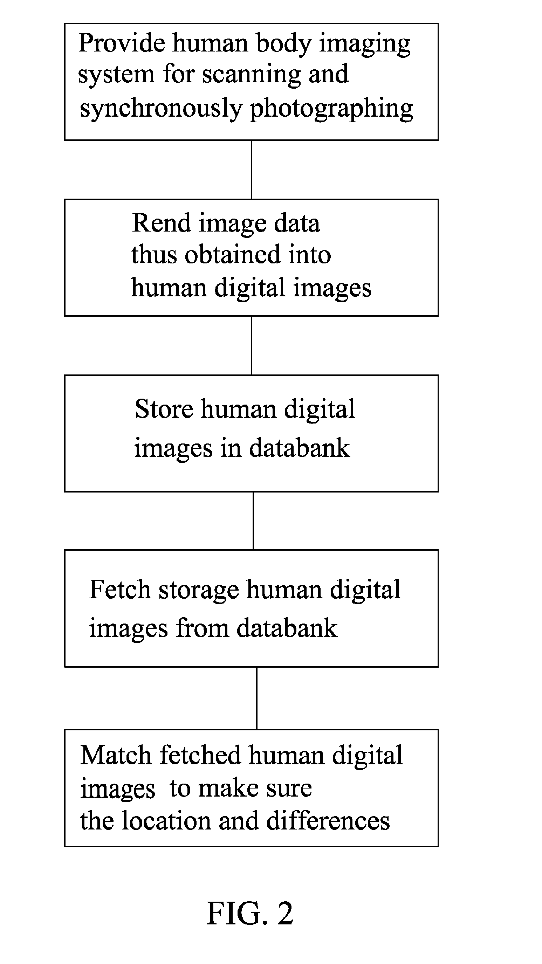

Referring to FIG. 2, a human body data matching method for medical cosmetic applications in accordance with the present invention includes the steps of:
a) providing a human body imaging system comprising a digital image pickup module for scanning and photographing the human bodies of clients synchronously based on a reference point to obtain image data of the soft tissues and hard tissues and skin tone appearance of predetermined parts of the physical structure of the human bodies;
b) rendering the image data thus obtained into human digital images;
c) storing the human digital images in a databank;
d) fetching pre-stored storage human digital images of the patient going to take an orthopedic or cosmetic surgery from the databank for future reconstruction or study of how individual growth pattern, or for forensic or recreational purposes prior to performing the orthopedic or cosmetic surgery; and
e) matching the fetched human digital images to make sure the location and differences.

In application, use the human body imaging system to scan and photography the human body of every client during his(her) best human body lifetime, for example, 18-year-old to 21-year-old, thereby obtaining the related image data, and then render the image data thus obtained into digital images. The scanned image data includes the data of the skeleton and tissues of the physical structure of the client's human body. The photographed image data includes the data of the skin tone appearance of the physical structure of the client's human body. Thereafter, store the digital images in a databank permanently subject to the client's personal information, such as date of birth, name and personal ID. When a client needs to take an orthopedic or cosmetic surgery in the future, the surgeon can fetch this client's storage digital images from the databank for matching and reference prior to performing the surgery.

Because human teeth will start to wear down, human skin tone appearance will change and human skeleton will shrink when people are getting old, fetching the storage digital images from the databank for matching and reference prior to performing a surgery can prevent errors in communication with the surgeon, assuring surgery satisfaction.

For example, when going to receive a skin whitening treatment, the skin tone appearance-related storage digital images can be fetched from the databank for reference so as to achieve a satisfactory skin whitening treating result.

Further, the invention can also be used for dental applications, allowing the dentist to fine the patient's (client's) original lower jaw height subject to the related storage digital images prior to dental implant, and thus, optimal dental implant can be achieved.

Further, prior to performing a knee joint replacement surgery to treat an injured knee joint, the surgeon can fetch the early stored digital images of the knee joint of the patient (client) from the databank for matching and reference, ensuring optimal surgical results.

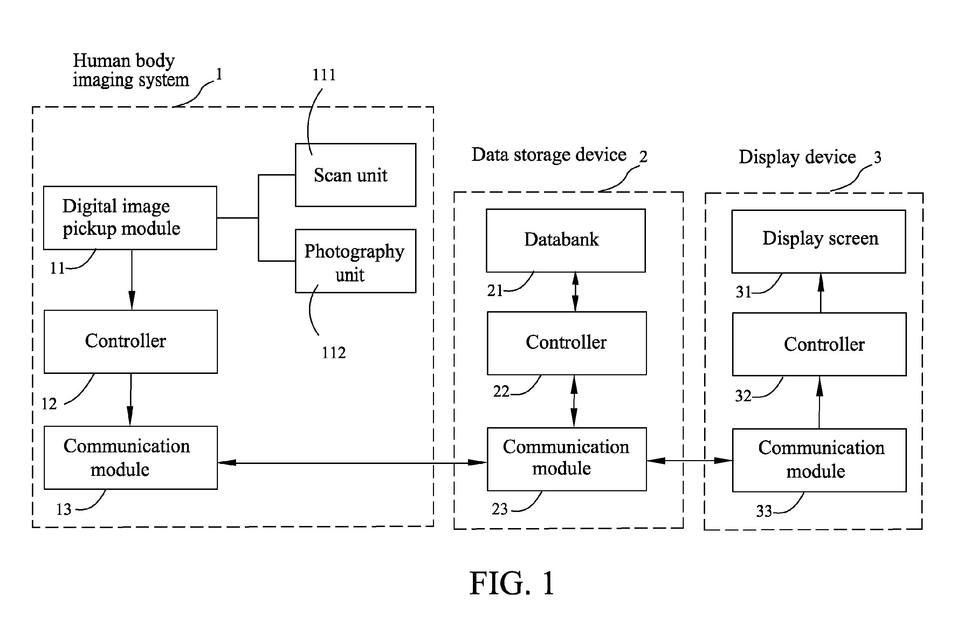

For the application of the aforesaid human body data matching method, the invention also provides a digital image storage system. As illustrated in FIG. 1, the digital image storage system comprises a human body imaging system **1,** a data storage device **2,** and a display device **3.**

The human body imaging system **1** comprises a digital image pickup module **11,** a controller **12** and a communication module **13.** The digital image pickup module **11** comprises a scan unit **111** having at least one of the functions of 2D and 3D full body scan, magnetic resonance imaging (MRI), ultrasound imaging, computer tomography and intra and extra oral scan, and a photography unit **112** that is a color camera capable of taking color pictures. The digital image pickup module **11** is controlled to scan and photograph the human bodies of clients during their optimal lifetime synchronously based on a reference point to obtain image data of the soft tissues and hard tissues of predetermined parts of the physical structure of the human bodies and the related skin tone appearance, and to render the image data thus obtained into human digital images. The human digital images are stored in the data storage device **2,** and can be displayed on the display device **3.**

In the future, pre-stored storage human digital images can be fetched from the data storage device **2** and displayed on the display device **3** for reference in a future reconstruction or study of how individual growth pattern, or for forensic or recreational purposes.

Further, the controller **12** of the human body imaging system **1** can convert the digital images into a predetermined format for transmission by the communication module **13** to the data storage device **2.**

The data storage device **2** comprises a communication module **23** adapted for receiving the formatted digital image data from the communication module **13** of the human body imaging system **1** for enabling the received digital image data to be stored in the databank **21** subject to the control of the controller **22.** The communication module **23** can also be controlled by the controller **22** to transmit storage digital image data to the display device **3** for display.

The display device **3** comprises a display screen **31,** a controller **32,** and a communication module **33** for receiving digital image data from the communication module **23** of the data storage device **2** for display on the display screen **31** subject to the control of the controller **32.**

Although a particular embodiment of the invention has been described in detail for purposes of illustration, various modifications and enhancements may be made without departing from the spirit and scope of the invention. Accordingly, the invention is not to be limited except as by the appended claims.

## Claims

1. A digital image storage system, comprising:
a human body imaging system (1) comprising a digital image pickup module (11) for scanning and photographing the human bodies of persons during their optimal lifetime synchronously based on a reference point to obtain image data of the soft tissues and hard tissues of predetermined parts of the physical structure of the human bodies and the related skin tone appearance and to render the image data thus obtained into human digital images;
a data storage device (2) electrically connected to said human body imaging system (1) for receiving and storing said human digital images; and
a display device (3) electrically connected to said data storage device (2) for displaying said human digital images.

2. The digital image storage system as claimed in claim 1, wherein said digital image pickup module (11) comprises a scan unit (111) and a photography unit (112), said scan unit (111) providing at least one of the functions of 2D and 3D full body scan, magnetic resonance imaging (MRI), ultrasound imaging, computer tomography and intra and extra oral scan.

3. The digital image storage system as claimed in claim 2, wherein said photography unit (112) comprises a color camera for taking color pictures.

4. The digital image storage system as claimed in claim 1, wherein said human body imaging system (1) further comprises a controller (12) and a communication module (13) electrically connected to said digital image pickup module (11) for converting said digital images into a predetermined format and transmitting the formatted digital image data to said data storage device (2).

5. The digital image storage system as claimed in claim 1, wherein said data storage device (2) comprises a databank (21) for storing digital image data, a controller (22), and a communication module (23) adapted for receiving digital image data from said human body imaging system (1) for enabling received digital image data to be stored in said databank (21) subject to the control of the controller of said data storage device (2).

6. The digital image storage system as claimed in claim 1, wherein said display device (3) comprises a display screen (31) for displaying digital image data, a controller (32), and a communication module (33) for receiving digital image data from said data storage device (2) for display on said display screen (31) subject to the control of the controller of said display device.

7. A human body data matching method for medical cosmetic applications, comprising the steps of:
a) providing a human body imaging system (1) comprising a digital image pickup module (11) for scanning and photographing the human bodies of predetermined persons synchronously based on a reference point to obtain image data of the soft tissues and hard tissues and skin tone appearance of predetermined parts of the physical structure of the human bodies of said predetermined persons;
b) rendering the image data thus obtained into human digital images;
c) storing said human digital images in a databank (21);
d) fetching pre-stored storage human digital images of one said predetermined person going to take an orthopedic or cosmetic surgery from said databank (21); and
e) matching the fetched human digital images to make sure the location and differences.
